# EUROPEAN PATENT APPLICATION

(11) **EP 4 253 527 A1**
(43) Date of publication of application: **04.10.2023**
(21) Application number: 22165448.6
(22) Date of filing: 30.03.2022
(51) Int. Cl.: C12N 5/00, B82Y 30/00

(54) **COATED ARTICLE FOR CULTURING PRIMARY CELLS AND STEM CELLS AND METHOD FOR PREPARING THE SAME**

(71) Applicant: Denovomatrix GmbH, 01307 Dresden (DE)
(72) Inventor: Dr. HENDEL, Thomas, 01099 Dresden (DE); Dr. SEGELETZ, Sandra, 01097 Dresden (DE); Dr. THAMM, Kristina, 01097 Dresden (DE); Dr. WETZEL, Richard, 01309 Dresden (DE)
(74) Representative: Maikowski & Ninnemann Patentanwälte Partnerschaft mbB

(57) **Abstract**

The invention relates to a coated article for culturing primary cells and stem cells, said coated article comprising a cell culture scaffold material with a non-covalent coating, said non-covalent coating comprises or consists of one or more layers selected from a layer consisting of at least one negatively charged polysaccharide (NCP), a layer consisting of a positively charged conjugate and optionally a layer comprising or consisting of a charge-inducing component, wherein said charge-inducing component of said primer layer is selected from poly-L-lysine, poly-D-lysine, poly-ornithine, poly(allylamine hydrochloride) (PAH), polydimethyldiallyl ammonium chloride (PDDA), and positively charged polypeptides. The invention further relates to a method of preparing said coated article and its use in regenerative, aesthetic medicine, and in cellular agriculture.

## Description

### Field of the Invention

The invention relates to a coated article for culturing primary cells and stem cells, said coated article comprising a cell culture scaffold material with a non-covalent coating, said non-covalent coating comprises or consists of one or more layers selected from a layer consisting of at least one negatively charged polysaccharide (NCP), a layer consisting of a positively charged conjugate and optionally a layer comprising or consisting of a charge-inducing component, wherein said charge-inducing component of said primer layer is selected from poly-L-lysine, poly-D-lysine, poly-ornithine, poly(allylamine hydrochloride) (PAH), polydimethyldiallyl ammonium chloride (PDDA), and positively charged polypeptides. The invention further relates to a method of preparing said coated article and its use in regenerative, aesthetic medicine, and in cellular agriculture.

### Background of the Invention

The industrial-scale cultivation of human cells for medicinal products and of animal derived cells for cellular agriculture is an outstanding biological and logistic problem. Research-scale cell cultivation is currently realized by growing cells on 2-dimensional (2D) cell culture plates/stacks. Industrial-scale cell cultivation requires more cost-effective culture formats with increased process control and reduced human interactions such as bioreactors. Within bioreactors cells are cultured in 3D as aggregates or on scaffolds such as microcarriers, hollow fibers, or sponge-like materials. A variety of materials have been used to produce these scaffolds, such as polystyrene, dextran, poly-lactic-glycolic acid (PLGA), alginate, collagen, gelatin, silk in both bulk and porous structures¹⁻³. Applying such materials for anchorage-dependent cells requires further modifications. Anchorage-dependent cells like embryonic, adult or induced stem cells, progenitor cells, and terminally differentiated primary cells strictly rely on adhesion ligands derived from their natural extracellular matrix (ECM) for their growth and survival. Therefore, 2D and 3D surfaces are routinely coated with substrates that aim to recreate the essential cues of the cellular microenvironment necessary for their growth. There are a variety of biological, regulatory, and economic constraints on the nature of those coating materials, their manufacturing, their production, and on the coating procedure itself for their use in cellular agriculture or regenerative medicine. In addition, the development of culturing procedures for cells used in regenerative medicine and cellular agriculture is usually established in small-scale experiments on 2D surfaces (<150 cm²). As a consequence, the upscaling from 2D processes into 3D processes is necessary for all experimental procedures including ECM coatings which is not always possible. Therefore, most of the current coating materials are limited in their application for industrial-scale cultivation of mammalian cells.

One of the most frequently applied scaffolds for industrial cell manufacturing are microcarriers. Microcarriers provide large surface areas to support the growth and attachment of cells and can be implemented in stirred-tank bioreactors at industrial scale. Therefore, the use of microcarriers has been established since the 1980's in biopharmaceutical industry to produce vaccines and recombinant proteins by mammalian cell lines. Growth and maintenance of cells in an industrial-scale bioreactor is less laborious and economically more feasible than growing cells on 2D surfaces and allows constant monitoring of the culture conditions and streamlined processes. The culture of anchorage-dependent stem cells, progenitor cells, and different primary cells requires specific adhesion ligands and cannot be grown on untreated microcarriers. Microcarriers have been chemically modified to introduce functional charged groups for supporting cell adhesion⁴. However, for most cell types either the initial adhesion is not sufficient to initiate cell growth or cell adhesion is too strong and cell harvest is limited on such microcarriers. Therefore, microcarriers are modified with ECM substrates or proteins or peptides to support the adhesion of those cell types.

Since the first application of cancer tissue extracts in 1986 by Kleinman and colleagues for cell culture, multiple ECM substrates have been developed with Matrigel being the most used in primary and stem cell cultures^{5,6}. Most of these substrates are not eligible for regenerative medicine or cellular agriculture due to their poor chemical definition and animal origin. However, proteins and peptides produced in vitro can be used in such applications. Coatings based on these components aim to recreate the minimal essential cues of the cellular microenvironment necessary for cell growth. ECM-proteins like fibronectin, vitronectin, and laminin are self-assembling and coat suitable surfaces by charge-related adsorption⁷. This approach is simple and avoids chemical crosslinking for stable immobilization of proteins on the surfaces which is beneficial for industrial processes.

Culture coatings made of full-length proteins are challenging to implement into industrial processes. Proteins are prone to denature and degenerate under ambient storage conditions and thus, tend to be complex and demanding in terms of storage (short shelf-life, low-temperature storage) or require *in situ* coating procedures. To overcome these problems, peptide-based coatings are fully chemically defined, easy to scale in production, and more robust for storage and therefore, provide a powerful alternative.

Peptide substrates are often applied to solely support the integrin-binding of the cells⁸. This minimal approach does not reflect the full complexity and functionality of the natural ECM⁹. The ECM also contains polysaccharides that provide mechanical support, serve as an aqueous reservoir, and aid in cell proliferation and function in addition to cell binding¹⁰⁻¹². To address cell-type-specific needs, it is important to use coating compositions that fulfill industry standards as well as the cell's biological requirements.

The use of cultured cells for/in humans also poses regulatory requirements on cell culture coatings. ECM extracts, as well as recombinant proteins, exhibit batch-to-batch variability due to their nature, which hinders reliable outcomes. Potential impurities and viral or prion contamination represent an additional risk and inhibit their application in the context of regenerative medicine, e.g. cell therapies. Alternative synthetic coatings often rely on covalent surface modifications that change the properties of the microcarrier raw material, which requires renewal of regulatory approval of a cell therapy process. Finally, like other ancillary materials for cell culture, coating materials need to be obtainable at pharma- or food-grade quality and at bulk quantity.

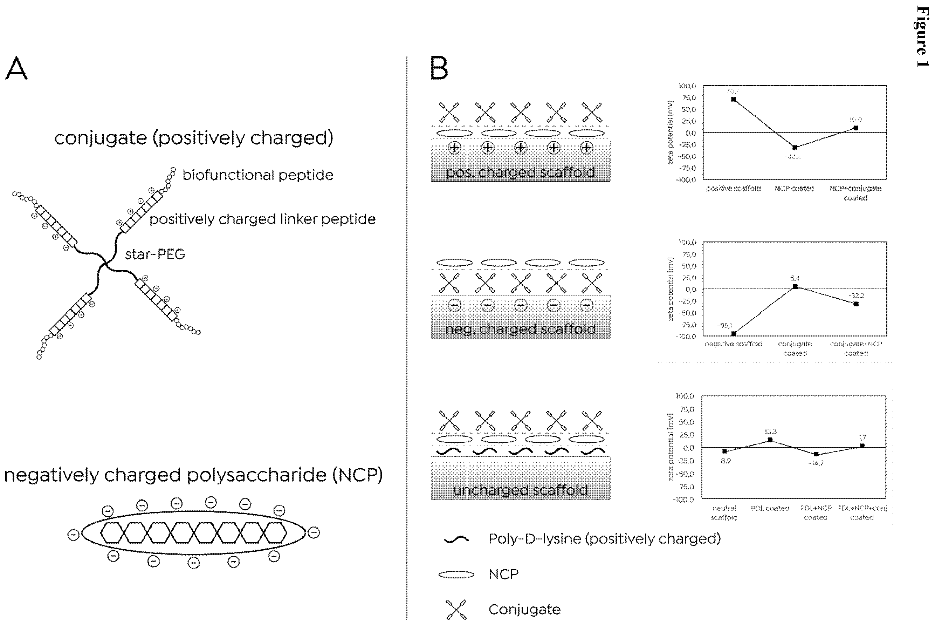

To address the plethora of demands on the nature and application of coatings culture of primary cells and stem cells, denovoMATRIX has previously invented a technology using polysaccharides and polymers conjugated with specific peptide sequences to form a non-covalent hydrogel by a coacervation-based method¹³. The formation of the hydrogel is driven by self-assembly and phase separation in aqueous solution (coacervation) followed by gravity-based deposition (Figure 1A). This approach has been demonstrated to enable the formation of stable bulk hydrogels for cell culture, cell encapsulation and bio-engineering tasks.

Based on this invention, a method has been developed to facilitate surface coating applications of cell culture articles. The coating application was reported to support serum-free culture of mesenchymal stromal cells useable for regenerative medicine^{14,15}. However, the gravity dependency of this method is limiting its application to planar surfaces (2D surfaces).

Further, the application of this coating procedure at industrial scale is limited by i) the sedimentation time of the formed coacervates from solution to the surfaces and *ii)* the high consumption of the coating materials used. Both aspects make the procedure uneconomic, which makes the procedure uneconomic. Layers prepared with this conventional approach have usually a thickness in the µm range, typically about 10 µm.

A layer-by-layer based coating method can overcome these limitations. Thomas et al. have shown that a layer-by-layer method can be applied using the polysaccharide heparin and starPEG-peptide-conjugates to create surface coatings with a linear growth pattern¹⁶. The reported method overcomes the drawbacks of the coacervation method by focusing on molecular charge interaction in a proof-of-principle study. However, the reported method has limited potential for application in cell culture industry. One aspect is the necessity of covalent coupling of the founding layer (in this example heparin has been used) to a glass support by silanization chemistry. This restricts its industrial application in two ways. First, covalent coupling in wet chemistry introduces an unfavorable complexity and additional time constrains, which are to be avoided for industrial processes. Second and most important, the method is restricted to SiO2-based materials like glass whereas most cell culture scaffolds are based on natural or synthetic polymers like dextran and polystyrene. In another aspect, the report demonstrates the principal suitability of the layer-by-layer approach to create a coating for cell culture. However, the employed cell model of fibroblast does not sufficiently demonstrate the eligibility of the reported formulation of more demanding cell types like primary cells and stem cells.

Purpose of the present invention has been to overcome the obstacles of the prior art and to provide a universal coating for cell-culture-relevant scaffold materials of different types, in particular 3D-scaffolds, preferably microcarriers for culture of mammalian cells, in particular primary cells, stem cells and cells derived thereof.

### Description of the invention

The present invention addresses the limitations of the prior art by focusing on a non-covalent purely charged-based coating that can be modified towards the various surface properties of cell culture scaffold materials. The invention is further addressing the requirements of adherent primary cells and stem cells in effective manner by integration of specific peptide sequences and optimized coating conditions.

The previously described limitations that only 2D surfaces can be coated are solved with this invention by applying a stepwise coating with several components in a layer-by-layer method wherein the coating is driven by adsorption und charge interaction.

In a preferred embodiment, the invention provides a coated article for culturing primary cells and stem cells, said coated article comprising a cell culture scaffold material with a non-covalent coating, said non-covalent coating comprises or consists of one or more layers selected from a layer consisting of at least one negatively charged polysaccharide (NCP), a layer consisting of a positively charged conjugate and optionally a layer comprising or consisting of a charge-inducing component.

Said cell culture scaffold material usually comprises a negatively charged surface, a neutral (i.e. uncharged) surface or a positively charged surface.

The charge of the surface of the cell culture scaffold material can be measured with conventional methods known to the person skilled in the art. A suitable method for determining the surface charge of the cell culture scaffold material is measuring the zeta potential at the surface when the cell culture scaffold material is selected from spherical 3D-particles with a particle size below 100 µm. When the cell culture scaffold material is a spherical 3D-particle with a particle size larger than 100 µm or a scaffold is a 2D-material, the surface coating can be monitored by fluorescence microcopy, e.g. as described in the examples of the invention. These methods are also applicable when the cell culture scaffold material is porous.

Zeta potential is a scientific term for the electrokinetic potential on the surface of a dispersed colloid. Around every colloidal particle dispersed in a solution an electrical double layer is formed due to charges on the particle surface affecting the distribution of ions at the interface with the surrounding solution. Brownian motion of the particle results in shearing forces which remove loosely bound ions while the inner firmly bound ions remain with the particle. The resulting electrical potential at the interface is defined as zeta potential and is a proportional measure for the otherwise unmeasurable net surface charge of the particle.

In the practical sense of the present invention, the surface of the cell culture scaffold material comprised in the coated article of the invention is negatively charged when the value of zeta potential is below -10 mV, for example between -250 mV and -10 mV. The surface of the cell culture scaffold material comprised in the coated article of the invention is neutral (i.e. uncharged), when the value of zeta potential is between -10 mV and 10 mV. The surface of the cell culture scaffold material comprised in the coated article of the invention is positively charged, when the value of zeta potential is higher than 10 mV, for example between 10 mV and 250 mV.

The finally coated article, i.e. after application of certain coating layers, shows a surface charge in the range between -50 mV and 50 mV, preferably between -30 mV and 30 mV, more preferably between -10 mV and 10 mV, most preferably between -3 mV and 3 mV. A suitable method for determining the surface charge of the finally coated article is measuring its zeta potential. The method is described in the examples of the invention.

Another possibility to estimate the charge of the finally coated article is the use of fluorescence imaging. As an example, microcarriers used for fluorescent imaging are coated using the layer-by-layer technique. All coatings may e.g. contain 5% of a Cy5-fluorescently labeled conjugate and the NCP contained 50% of a FITC-fluorescently labeled NCP. Fluorescence imaging is e.g. carried out using a Lionheart FX automated microscope (Agilent) and settings are kept equally in each experiment. Fluorescence intensity can be quantified using the Gen5 software (BioTek) Version 3.10.06 and statistical processing performed using the GraphPad Prism software version 9.3.1. The determined fluorescence intensity can be used to quantify the charge of the finally coated article indirectly.

Surprisingly, it has been found in the examples of the present invention that the change of the zeta potential by application of certain coating layers indicates the effectiveness of the surface coating and predicts the adherence or binding of cells to the coated article. Positive effects on the adherence or binding of cells to the coated article have been found, when the surface of the coated article shows a zeta potential in the range between -50 mV and 50 mV, preferably between -30 mV and 30 mV, more preferably between -10 mV and 10 mV, most preferably between -3 mV and 3 mV.

Charged surfaces enable a strong interaction with cell surfaces. Therefore, the surface charge of cell culture scaffolds is conventionally enhanced by chemical modification to promote binding of the cells to these articles. Is has been applied by the industry as an effective strategy to enable the culture of human and animal derived cells. However, this procedure has the disadvantage that increased surface charges of the cell culture scaffolds promote an unspecific interaction with the cell surfaces rather than a specific cell binding.

The LbL coating procedure of this invention is based on interaction of charged polymers with the charged scaffold surfaces which leads to a reduction of the absolute value of the charge. It has been surprisingly found that, although the surface charge of the coated article is lowered by the LbL coating procedure in the range as described above, most preferably in the range of -30 mV to 30 mV, the cell binding can be even more increased, and specificity of the cell binding is enhanced and/or can be controlled.

Surface charge is not the dominating criteria and moreover, not the most beneficial to culture high quality stem cells. The stem cells are provided with essential mechanical cues of being bound to a rigid interface but bioactive signals reassembling their niche of origin are missing. Furthermore, detachment and harvest of unspecifically bound cells require harsh, potentially quality-impacting conditions with conventional coatings. This disadvantage can be overcome with the LbL coating of the present invention.

It is shown that besides the zeta-potential remained unchanged with increased concentrations of coating materials, cultured induced pluripotent stem cells prefer high concentration (e.g. between 5 µM and 50 µM) of the coating materials.

The presence, concentration, and homogeneous distribution of biofunctional ligands on the scaffold supports efficient cell attachment and growth in a biologically relevant manner by stimulation of adhesion receptors. In the given invention, the specific ligand-receptor binding is promoted by the bioactive peptides comprised in the coating which are responsible for cell binding to the coated article. This is advantageous because it supports cell expansion while maintaining expression of the expected cell morphology, and stem cell identity. It further enables an enzymatic detachment of the cells from the surface of the coated article proving the low impact on the cells as shown by multi-passage experiments.

In contrast, in the case of a resulting surface charge of the coated article outside the range described above, i.e. in a deep negative or high positive range, the formation of protein-protein-interactions (receptor binding) between surface proteins of the cells and the bioactive peptides comprised in the coating layers is disturbed and charge-charge-interactions, such as ionic bindings, are dominating the cell binding to the coated article. This is disadvantageous, because it prevents an easy detachment of the cells from the surface of the coated article and cells would rather be damaged or destroyed during their removal.

Measurements of the surface charge, such as zeta potential measurements are a powerful tool to demonstrate the effective deposition of these biofunctional materials on the coated scaffold. Significant alternations of the zeta potential (Δ ≥ 20 mV) upon each coating step proof the efficiency of deposition of each layer.

It is a special advantage of the invention that cell culture scaffold material, independent from its surface charge, can be coated such that cells can adhere or bind to the coating and that the coated article can be used for cell culturing. Accordingly, a great variety of cell culture scaffold materials can be used within the invention.

Suitably, the cell culture scaffold material is selected from synthetic polymers such as polystyrene and poly-lactic-glycolic acid (PLGA), and natural polymers such as polysaccharide like dextran, starch, alginate, pectin, proteinergic materials like silk, soy protein, collagen, and gelatin. Any of these materials can be positively charged, negatively charged and neutral depending on their production conditions. Moreover, they can be used in 2D-shape or 3D-shape and as non-porous or porous material or as hollow fiber material. Preferred, according to the invention, is 3D-cell culture scaffold material, in particular microcarriers. A special advantage of the invention is that 3D-materials can be coated because the coating is gravity-independent and is instead driven by diffusion, adsorption und charge interaction, in particular electrostatic interaction.

A non-covalent interaction differs from a covalent bond in that it does not form molecular orbitals, but rather involves more dispersed variations of electromagnetic interactions between molecules or within a molecule. Commonly, non-covalent interactions are considered weaker than covalent bonds, i.e. non-covalent interactions have lower dissociation energies. Typical non-covalent interactions include electrostatic interactions such as polar interactions, hydrogen bonding, halogen bonding, Van der Waals forces including dipole-dipole, and induced dipol interactions as well as London dispersion forces, and π effects like π-π interactions, cation-π and anion-π interactions, as well as hydrophobic effects. All these effects are known to the person skilled in the art.

The coated article of the invention is suitably produced by the so-called layer-by layer (LbL) coating, which is described herein below. Depending on the charge of the surface of the cell culture scaffold material, the order of the layers coated onto the scaffold material is different.

In a preferred embodiment the invention provides a coated article according to any one of the preceding claims, wherein said cell culture scaffold material comprises a negatively charged surface or positively charged surface, which comprises a multilayer coating comprising or consisting of the structure (YZ)ₙ or (YZY)ₙ for the negatively charged scaffold surface; or (ZY)ₙ or (ZYZ)ₙ for a positively charged surface, wherein
Y represents a layer comprising or consisting of a positively charged conjugate;
Z represents a layer comprising or consisting of at last one negatively charged polysaccharide (NCP); and
n is an integer in the range of 1 and 100.

Preferably, n is an integer in the range of 1 to 90, 1 to 80, 1 to 70, 1 to 60, more preferably 1 to 50, or 1 to 40 or 1 to 30, most preferably 1 to 20, 1 to 10 or 1 to 5.

"Structure" means order of the layers relative to the surface of the cell culture scaffold material. For example, in the structure (YZ)ₙ, layer Y is coated onto the surface of the cell culture scaffold material as first layer, and layer Z is subsequently coated onto layer Y. In the structure (ZY)ₙ, layer Z is coated onto the surface of the cell culture scaffold material as first layer, and layer Y is subsequently coated onto layer Z.

The layer-by-layer coating of the present invention is especially suitable for the coating of 3D-scaffolds. Most preferred according to invention is therefore a coated article, wherein said cell culture scaffold material has a three-dimensional structure, such as a microcarrier. It may be porous or not.

A further advantage of the layer-by-layer coating of the present invention is that the thickness of the layers is very low compared to other coating approaches known in the prior art. Preferably, the typical thickness of one layer is 10 nm or lower, preferably <10 nm, more preferably 9, 8, 7, 6, 5, 4, 3, 2 or 1 nm. This leads to a material saving of 100 to 1,000-fold compared to conventional coating approaches and gives the layer-by-layer coating of the present invention an economic advantage.

Layer Y has two functions: It is positively charged to interact with a negatively charged surface of the cell culture scaffold material or with the negatively charged layer Z via charge interactions or adsorption. In order to enable the adhering or binding of cells to the coated article of the invention, layer Y further comprises means for cell binding or interaction with cells.

In a more preferred embodiment, layer Y comprises or consist of a polymer wherein a peptide sequence is coupled to it to create a conjugate, and wherein said peptide sequence consists of a linker peptide (KA)ₙ and a biofunctional peptide. In said (KA)ₙ, K is lysine, A is alanine and n is an integer selected from 3, 4, 5, 6, 7, 8, 9, 10, 11, and 12. In preferred embodiments, n is 5, 7 or 9. So, preferably, the linker peptide is selected from (KA)₅ (SEQ ID NO. 1), (KA)₇ (SEQ ID NO. 2) and (KA)₉ (SEQ ID NO. 3). Most preferably, the linker peptide is (KA)₇ (SEQ ID NO. 2). The linker peptide is suitably positively charged and adds the positive charge to the entire conjugate of layer Y.

In the peptides of the present invention, each amino acid residue is represented by a one-letter or a three-letter designation, corresponding to the trivial name of the amino acid, in accordance with the following conventional list (Table 1):

**Table 1. Designation of amino acid residues**

| Amino Acid | **One-Letter Symbol** | **Three-Letter Symbol** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

Said biofunctional peptide is capable of interaction with cells and enables their binding to the coated article of the invention. The biofunctional peptide is preferably selected from the peptides of SEQ ID NOs: 4 to 344. These sequences comprise oligopeptide sequences which mimic the ECM and signal peptides. More preferably, the biofunctional peptide is one of those shown in Table 2. These sequences are representing binding sites from proteins of the extracellular matrix, which are most relevant for primary and stem cells, e.g., laminin, fibronectin, vitronectin, bone sial protein, collagen, and tenascin.

**Table 2. List of most preferred peptide sequences.**

| **Biofunctional Peptide** | **SEQ ID NO** |
|---|---|
| KA5 | 1 |
| KA7 | 2 |
| KA9 | 3 |
| RGDSP | 4 |
| FRLVFRY | 5 |
| NGEPRGDTYRAY | 6 |
| NPWHSIYITRFG | 7 |
| PHSRN | 8 |
| PHSRNGGRGDSP | 9 |
| RGD | 10 |
| CSRNLSEIKLLISRARK | 11 |
| DVDVPDGRGD SLAYG | 12 |
| EIKLLIS | 13 |
| GEFYFDLRLKGDKY | 14 |
| GFPGER | 15 |
| LEPRREVCELNPD | 16 |
| LFSHAVSSN | 17 |
| LGGLPSHYRARNI | 18 |
| NRWHSIYITRFG | 19 |
| PQVTRGDVFTMP | 20 |
| RKIPKASSVPTELSAISMLYL | 21 |
| SIDRVEPYSSTAQ | 22 |
| TFDLLRNSYGVRK | 23 |
| TWSKVGGHLRPGIVQSG | 24 |
| TWYKIAFQRNRK | 25 |
| VAEIDGIELT | 26 |
| VFDNFVLK | 27 |
| QPPRARI | 28 |
| YRSRKYS SWYVALKRK | 29 |
| ESPLKRQ | 30 |

Most preferred according to the invention are the biofunctional peptides selected from the group consisting of SEQ ID NOs: 4, 5, 6, 7, 8 and 9. These peptide sequences are most important for the culture of stem cells like mesenchymal stem cells and induced pluripotent stem cells and cell types derived thereof and are of special interest for the application of this invention.

In a further preferred embodiment of the invention, the polymer used in layer Y is polyethylene glycol (PEG). PEG is an oligomer or polymer composed of ethylene oxide monomers. Because different applications require different polymer chain lengths, PEGs are prepared by polymerization of ethylene oxide and are commercially available over a wide range of molecular weights from 300 g/mol to 10,000,000 g/mol. While PEGs with different molecular weights find use in different applications, and have different physical properties (e.g. viscosity) due to chain length effects, their chemical properties are nearly identical.

PEGs are also available with different geometries:
- Linear PEGs, where the ethylene oxide monomers are bound to each other in an unbranched polymer chain;
- Branched PEGs, which have three to ten PEG chains emanating from a central core group;
- Star PEGs, which have 10 to 100 PEG chains emanating from a central core group; and
- Comb PEGs, which have multiple PEG chains normally grafted onto a polymer backbone.

The numbers that are often included in the names of PEGs indicate their average molecular weights (i.e. a PEG with n = 9 would have an average molecular weight of approximately 400 Da and would be labeled PEG 400). Most PEGs include molecules with a distribution of molecular weights (i.e. they are polydisperse). The size distribution can be characterized statistically by its weight average molecular weight (M_{w}) and its number average molecular weight (Mn), the ratio of which is called the polydispersity index (M_{w}/Mₙ). M_{w} and Mₙ can be measured by mass spectrometry.

PEG is soluble in water, methanol, ethanol, acetonitrile, benzene, and dichloromethane, and is insoluble in diethyl ether and hexane.

In a preferred embodiment, layer Y of the coated article of the invention comprises a starPEG. Suitably, said starPEG has a molecular weight in the range of 4 kDa to 40 kDa, preferably in the range of 4 kDa to 30 kDa, more preferably in the range of 4 kDa to 20 kDa, most preferably in the range of 4 kDa to 10 kDa. Further most preferably, said starPEG is a 4-arm starPEG. The use of 4-arm starPEG in the hydrogels according to the invention has been found to be especially suitable when a peptide sequence is coupled to each of the four arms, and when said peptide sequence consists of a linker peptide (KA)ₙ and a biofunctional peptide as described above.

More preferably, layer Y of the coated article of the invention comprises a linear PEG. Linear PEGs have the advantage of being cheaper and possess a narrower molecular weight distribution. The use of a linear PEG in layer Y of the coated article according to the invention has been found to be especially suitable, when a peptide sequence is coupled to each of both ends, and when said peptide sequence consists of a linker peptide (KA)ₙ and a biofunctional peptide as described above. By using linear PEG instead of starPEG, the charge density of the positive charges in the conjugate of layer Y is higher, i.e. the portion of the uncharged structure-giving polymer is lower, which saves material and costs.

Most preferably, the linear PEG comprised in layer Y of the coated article according to the invention has a molecular weight in the range of 1 kDa to 100 kDa, preferably in the range 2 kDa to 80 kDa, 3 kDa to 60 kDa, 4 kDa to 40 kDa, most preferably in the range of 5 kDa to 20 kDa. Even most preferably, the linear PEG comprised in the conjugate according to the invention has a molecular weight selected from 5 kDa, 10 kDa, 15 kDa and 20 kDa.

In a further preferred embodiment, the PEG, which is used to prepare layer Y of the coated article of the invention, is functionalized. It is meant by "functionalized" to modify a molecule in a manner that results in the attachment of a functional group or moiety. For example, a molecule may be functionalized by the introduction of a functional group or structural feature which makes the molecule a strong nucleophile or a conjugated unsaturation. Preferably a molecule, for example PEG, is functionalized to become a thiol, amine, acrylate, azide, alkyne, or quinone. More preferably, for use in the preparation of the conjugate of the invention, the PEG is maleimide-functionalized, carboxylic acid-functionalized, amino-functionalized, azide-functionalized, or alkyne-functionalized.

Said negatively charged polysaccharide (NCP) of layer Z interacts with the positively charged surface of the cell culture scaffold material or with the positively charged layer Y via charge interaction or adsorption and is preferably selected from poly(sodium-4-styrenesulfonate) (PSS) or a sulfated or phosphorylated oligosaccharide, preferably selected from a group consisting of polysaccharides which comprises heparin, heparan sulfate, chondroitin sulfate, dermatan sulfate and keratin sulfate, dextran sulfate, α-cyclodextrin sulfate, β-cyclodextrin sulfate, γ-cyclodextrin sulfate, α-cyclodextrin phosphate, β-cyclodextrin phosphate, and γ-cyclodextrin phosphate.

In an especially preferred embodiment the NCP is heparin, which originates from the mucosa of pig intestine or bovine lung tissue. Heparin is preferably of pharmaceutical quality. In an alternative embodiment the NCP is dextran sulfate, which preferably has a molecular weight in the range of 4 kDa to 600 kDa. Preferred is the use of dextran sulfate of pharmaceutical quality. If layer Z contains cyclodextrin sulfate, then it is preferably α-cyclodextrin sulfate, β-cyclodextrin sulfate, γ-cyclodextrin sulfate of pharmaceutical quality, wherein the sulphation degree is of three sulfates per molecule up to a complete sulphation degree. If layer Z contains α-cyclodextrin phosphate, β-cyclodextrin phosphate, γ-cyclodextrin phosphate then it is of pharmaceutical quality, wherein the degree of phosphorylation of three phosphate groups per molecule can be up to the complete phosphorylation.

In a further embodiment, the coated article of the invention may comprise a cell culture scaffold material with a neutral (i.e. uncharged) surface. In this case, the coating of the coated article may further comprise a primer layer comprising or consisting of a charge-inducing component, which adds a positive charge to the surface of the uncharged cell culture scaffold material for further coating with the layers Y and Z.

Accordingly, in a further preferred embodiment, the invention provides a coated article, wherein said cell culture scaffold material comprises a neutral (uncharged) surface, which comprises a coating comprising or consisting of:
i. a layer comprising or consisting of a charge-inducing component as primer layer; and
ii. a multilayer coating comprising or consisting of the structure (ZY)ₙ or (ZYZ)ₙ as defined herein above.

The charge-inducing component is preferably selected from poly-L-lysine, poly-D-lysine, poly-ornithine, poly(allylamine hydrochloride) (PAH), polydimethyldiallyl ammonium chloride (PDDA) or other positively charged polypeptides.

It is a special advantage of the invention that the coated article as described herein is obtainable by layer-by-layer coating, which is driven by charge-based interactions and/or adsorption between the surface of the cell culture scaffold material and the coating layers.

In a further embodiment, the coated article of the invention may comprise cells. The cells are typically eukaryotic cells, preferably from vertebrates and anthropoids, preferably mammalian cells, most preferably human cells, or human cell lines. In a more preferred embodiment, the cells are pluripotent stem cells especially embryonic and induced pluripotent stem cell (iPSC) and all cell types derived thereof, and all primary adult stem and progenitor cells especially satellite cells, bone marrow derived mesenchymal stem cells (bmMSC), umbilical cord derived mesenchymal stem cells (ucMSC), and adipose tissue derived mesenchymal stem cells (atMSC), and any cell type thereof, myoblasts and adipose progenitor cells, as well as primary somatic cells like fibroblasts, endothelial and epithelial cells. Most preferably, the cells are primary cells and stem cells.

Typically, the cells interact with the biofunctional peptide of the conjugate of layer Y and bind or adhere thereby to the coated article of the invention.

The biofunctional peptide can include an amino sequence derived from ECM proteins such as collagen, fibronectin, vitronectin, laminin, bone sialo protein, or the like, or portions thereof. In embodiments, the biofunctional peptide includes an amino acid sequence of Arg-Gly-Asp (RGD) or a derivative cell adhesive sequence, recognized by cell adhesion receptors like the integrin family or other molecules leading to cell adhesion.

In current cell culture practice, it is known that specific cell types require the presence of a specific adhesion peptide or combination of peptides on the culture surface to enable adhesion and sustainable culture. For example, iPSC prefer laminin or vitronectin derived peptide sequences such as the peptide NGEPRGDTYRAY of SEQ ID NO. 6. The invention further provides a method for preparing the coated article of the invention. It should be recognized that the advantages and advantageous embodiments described above for the coated article according to the invention equally apply to the method for preparing of said coated article such that it shall be referred to the above.

In particular, a method is provided for preparing a coated article comprising a cell culture scaffold material with a non-covalent coating according to any one of the preceding claims, comprising the steps of
i. selecting a cell culture scaffold material;
ii. measuring the charge (zeta potential) of the surface of the cell culture scaffold material, and
iii. depending on the charge of the surface of the cell culture scaffold, coating said cell culture scaffold with a multilayer coating comprising or consisting of the structure (YZ)ₙ or (YZY)ₙ for a negatively charged surface; or (ZY)ₙ or (ZYZ)ₙ for a positively charged surface, wherein
   Y represents a layer comprising or consisting of a positively charged conjugate;
   Z represents a layer comprising or consisting of at last one negatively charged polysaccharide (NCP); and
   n is an integer in the range of 1 and 100.

When said cell culture scaffold material comprises a neutral (uncharged) surface, it is coated with
a layer comprising or consisting of a charge-inducing component as primer layer; and a multilayer coating comprising or consisting of the structure (ZY)ₙ, or (ZYZ)ₙ as defined herein.

The method according to the invention is advantageous, because the layer-by-layer deposition on the cell culture scaffold material is driven by adsorption und/or charge interaction between the surface of the cell culture scaffold material and the coating layers. In particular, the method of the invention is gravity-independent and therefore suitable for coating of 3D-shaped structures.

The coating of 2D cell culture plastic ware and 3D scaffolds was performed using the LbL approach according to the invention. Diluted aqueous solutions (1 to 1,000 µM) of NCPs and positively charged conjugates in a suitable buffer, such as PBS buffer are prepared as coating solutions. Coating of plastic ware in 2D is performed by filling the well/flask with a first coating solution. In case of 3D scaffolds, samples have been immersed in the first coating solution and gentle swirling was applied. A duration of 30 to 60 min of coating duration is suitably applied. Thereafter, the materials are washed twice with water, preferably deionized water. Subsequently, the second coating solution is added, and the procedure is repeated likewise for every following coating step. Upon completion, coated articles were left for drying.

The present invention is based on a layer-by-Layer (LbL) coating, that presents a biologically functional improvement of existing solutions for use in industrial-scale cell manufacturing for regenerative medicine and cellular agriculture. The introduction of novel combinations of peptide sequences allows for culturing of sensitive anchorage-dependent cell types like stem cells. The simple coating protocol developed can be applied off- and online and used as off-the-shelf pre-coated products optimally suited for industrial processes. The novel coating protocol allows its application onto various types of material, in particular natural scaffolds, without further chemical modifications like crosslinking.

In a further step, cells may be adhered to the coated article by incubating the coated article in a culture medium comprising cells, preferably primary cells and/or stem cells, most preferably of human origin.

The invention further relates to the use of the coated article according to any one of the preceding claims in a method for culturing cells, preferably primary cells and/or stem cells, most preferably of human origin. In general, the coated article would also be suitable for the culturing of any type of eukaryotic cells.

In a further embodiment, the invention provides the coated article with cultured cells adhered to it for use in regenerative medicine and in cellular agriculture.

Regenerative and aesthetic medicine defines a medical treatment of any type of regeneration, supplementation, or replacement of cells, organs or tissues with cells, engineered cell-tissue or cultured organs or similar living therapeutics.

Cellular agriculture is defined as production of agriculture products from cell cultures derived from wild or domesticated animals.

In a further embodiment, the invention relates to the use of the coated article with cultured cells adhered to it for the preparation of a cellular therapeutic for the treatment of diseases or disorders in the field of regenerative medicine, like cardiac diseases e.g. hear failure, ophthalmic diseases e.g. retinal degenerations, macular degeneration/dystrophy, optic atrophy and retinopathy, ocular diseases; neurologic disorders e.g. Parkinson's disease, spinal cord injury, diabetes; blood diseases e.g. thalassemia, Sickle-cell disease; cancer.

In a further embodiment, the invention relates to the use of the coated article with cultured cells, preferably stem cells adhered to it in aesthetic dermatology and plastic surgery procedures, e.g. for application in dermatology (e.g. burns, scars, wrinkles, abnormalities) or plastic surgery to support wound healing process after filler injections, breast augmentation, or for supporting fat-grafting.

In a further embodiment, the invention relates to a method of treatment of diseases or disorders in the field of regenerative medicine comprising the administration of a therapeutically effective amount of the coated article with cultured cells adhered to it to a subject in need thereof.

In a further embodiment, the invention relates to cells derived from wild or domesticated animals of the myogenic lineage, e.g. myogenic progenitor, adipocytes, myoblast, satellite cells, skeletal muscle, cardiac muscle, smooth muscle as well as white adipocytes. In addition, the invention relates to MSCs and iPSCs and their derivatives of animal species origin, such as bovine, porcine, poultry, salmon, and tuna.

The invention is further illustrated by certain drawings and examples.

### Brief description of the drawings

- **Figure 1A)**: shows schematic sketches of the two main components used in the LbL approach. The positively charged conjugate consists of a star-PEG backbone conjugated to a linker peptide and a biofunctional peptide. The negatively charged polysaccharide (NCP) is a highly sulfated sugar molecule.
- **Figure 1B)**: shows schematic coating strategies for three differently charged scaffolds (positive, negative, neutral). Depending on the surface charge of the scaffold the coating order is adapted. Corresponding results from zeta potential measurements on a bead-based model system confirm as a prove of principle the effectiveness of each strategy.

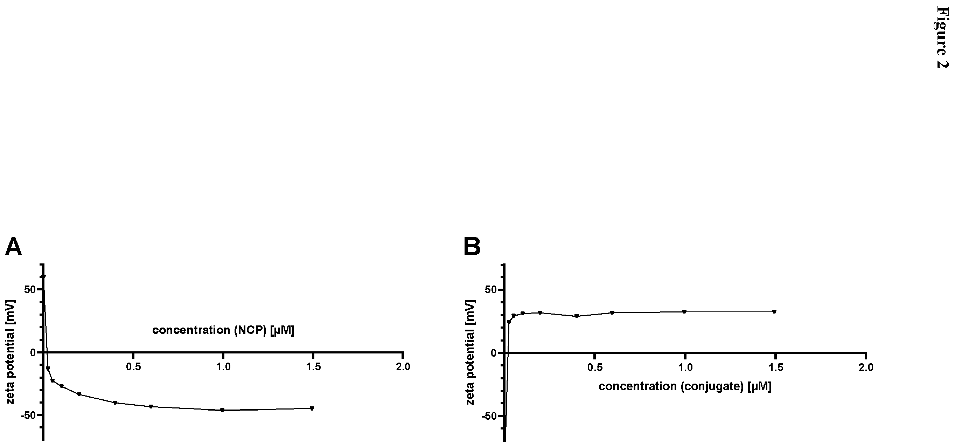

- **Figure 2**: shows the change of the zeta potential of different microcarriers upon stepwise addition of coating material. A) NCP is added stepwise to positively charged microcarriers. B) Conjugate is added stepwise to negatively charged microcarriers. In both cases, the zeta potential and thus, the surface charge changes significantly upon addition of coating material.

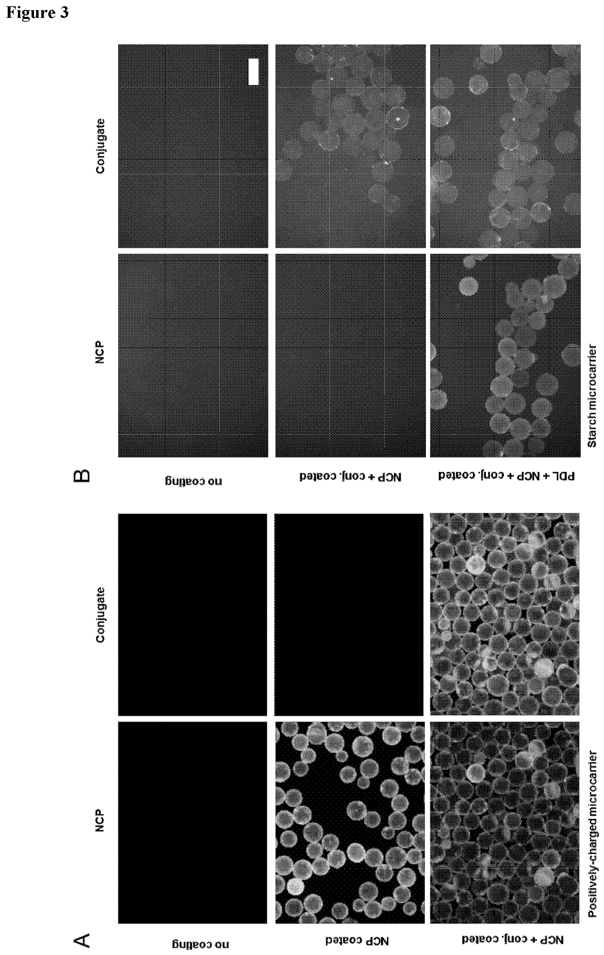

- **Figure 3**: shows that layer-by-layer coating is efficiently deposited on natural scaffolds. NPC and conjugate components are detected and displayed individually. A) Deposition of coating components onto a positively charged microcarrier. NCP is effectively deposited and detected in a first layer. Likewise, the conjugate is found in the second layer. B) Deposition of coating components on a natural, neutrally charged carrier composed of starch. Using only NCP and conjugate for the coating results in a poor coating efficiency. The addition of poly-D-lysine (PDL) aids in an efficient deposition by introducing positive charges. Scale bar: 100 µm.

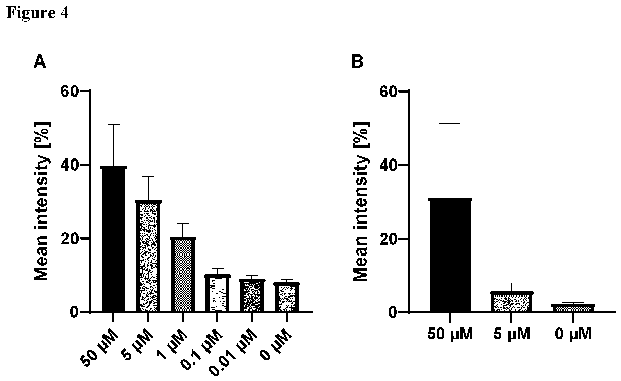

- **Figure 4**: shows the quantification of the amount of conjugate deposited on different microcarriers in dependency of the coating solution concentration. The coating solutions contain 5% of a fluorescently labelled conjugate and the fluorescence intensity is measured by wide-field fluorescence microscopy. A) Fluorescence intensity evolution of a positively charged microcarrier coated beforehand with NCP and subsequently coated with the conjugate. B) Fluorescence intensity evolution of a negatively charged microcarrier coated with the conjugate.

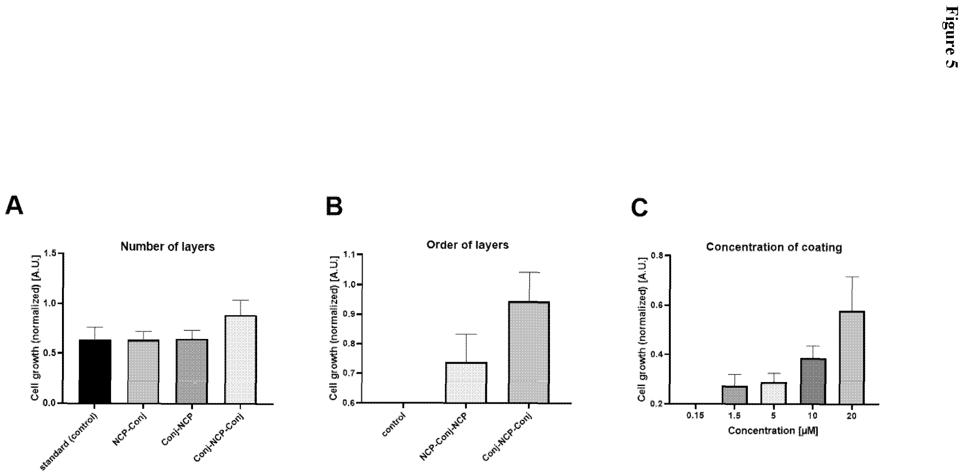

- **Figure 5**: shows that different parameters of layer-by-layer coating are tunable and contribute to optimizing cell response. Negatively charged polysaccharides (NPC) and positively charged conjugates (Conj) were deposited in different numbers of coating cycles, orders, and concentrations on 2D cell culture plates. A) Response of mesenchymal stromal cells (MSC) to different number of layers deposited in an LbL approach in comparison to a coacervation based control (n=3, tr=3). B) The impact of different orders of component deposition is shown for cell growth of MSC in comparison to a coacervation based control (n=3, tr=3). C) Cell growth of induced pluripotent stem cells (iPSCs) on layer-by-layer coatings with varying coating solution concentrations (n=2, tr=5).

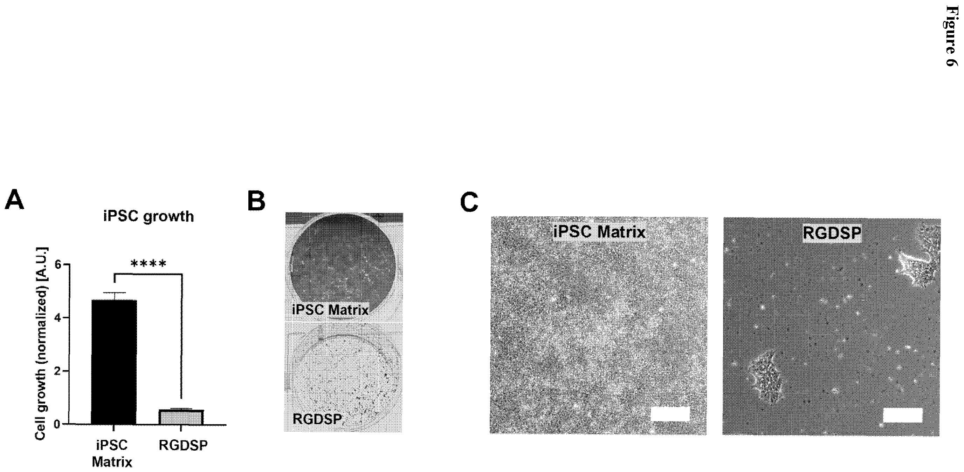

- **Figure 6**: shows that stem cells require specific coating formulations for optimal cell growth. A) Overall cell growth of iPSCs on plates coated LbL with either an optimized formulation (iPSC Matrix) or previously reported formulation (RGDSP) (n=3, p<0.0001. B) Visualization with crystal violet of cell growth on an entire coated well using iPSC Matrix or RGDSP formulation .C) Representative phase-contrast images of cell colonies grown on LbL coatings with either RGDSP or iPSC Matrix formulation.

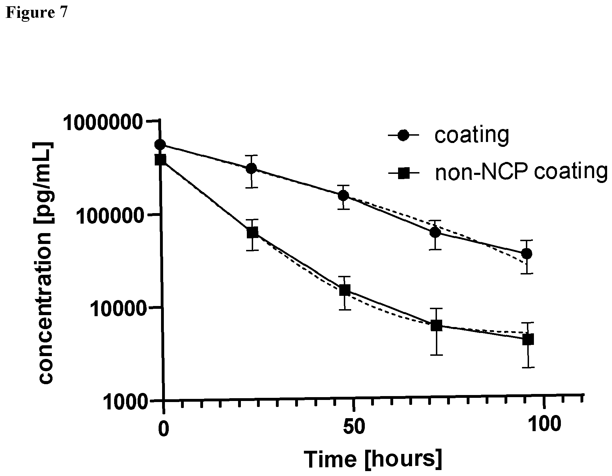

- **Figure 7**: shows the in vitro stabilization of FGF-2 by NCP coating component. mTESR1^{™} media relevant for iPSC growth was incubated at 37°C, 5% CO₂, for different periods on our coating and another animal-component free coating which does not contain NCP as a component. The concentration of FGF-2 present in the media was determined with an ELISA assay. The data was fitted using one phase decay model: Y=(Y0 - Plateau)^{∗}exp(-K^{∗}X) + Plateau, coating r²=0.905 and non-NCP coating r²=0.9914.

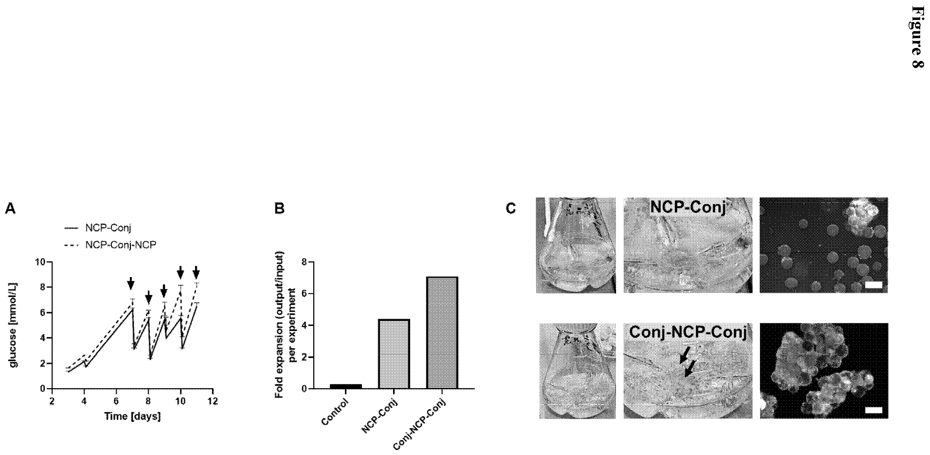

- **Figure 8**: shows that Lbl coating allows for 3D cultivation of mesenchymal stromal cells. A) Glucose consumption over time on microcarriers either coated with NCP-conj (2-layer) or NCP-conj-NCP (3-layer). Arrows indicate media change. B) Fold expansion of cell number seeded onto microcarriers with 2-layer or 3-layer coating after 11 days of cultivation. C) Images of cells cultivated on microcarriers in Erlenmeyer flasks. The formation of larger cell-microcarrier aggregates on 3-layer coating is highlighted in the magnified image and illustrated in the fluorescence microscope images. Scale bar: 100 µm.

### Examples of the Invention

### I. Material and Methods

### LbL coating procedure

Coating of 2D cell culture plasticware and 3D scaffolds was performed using the LbL approach. Dilute aqueous solutions (5-50 µM) of NCPs and conjugates in PBS buffer were used as coating solutions. Coating of plasticware in 2D was performed by filling the well/flask with the first coating solution. In the case of 3D scaffolds, samples have been immersed in the first coating solution and gentle swirling was applied. In both 2D and 3D, 30 to 60 min of coating duration was applied. Afterwards, samples were washed twice with deionized water. Subsequently, the second coating solution was added, and the procedure was repeated likewise for every following coating step. Upon completion samples were left for drying.

### Zeta potential measurements

Zeta potential measurements were carried out on a Zetasizer Nano ZS (Malvern Panalytical). Positively and negatively charged polystyrene beads were purchased from Polysciences Inc. Neutral beads were supplied by Surflay Nanotec GmbH. For zeta potential measurements, beads were suspended in dilute NaCl solutions (c = 1 mM) and suspensions were filled into Folded Capillary Zeta Cells (Malvern Panalytical). The electrophoretic mobility of the particles is measured using a laser Doppler velocimeter. Zeta potential values were approximated using the Smoluchowski model included in the Zetasizer Nano software (Malvern Panalytical). For the coating titration experiments, positively and negatively charged polystyrene beads were coated with solutions of NCPs and conjugates in dilute aqueous NaCl solutions (c = 1 mM) instead of PBS buffer.

### Fluorescent imaging of coated microcarriers

Microcarriers used for fluorescent imaging were coated using the layer-by-layer technique. All coatings contained 5% of a Cy5-fluorescently labeled conjugate and the NCP contained 50% of a FITC-fluorescently labeled NCP. Fluorescence imaging was carried out using a Lionheart FX automated microscope (Agilent) and settings were kept equally in each experiment. Fluorescence intensity was quantified using the Gen5 software (BioTek) Version 3.10.06 and statistical processing performed using the GraphPad Prism software version 9.3.1.

### Growth experiments for coating optimization

Human iPSCs were cultured in mTeSR^{™}1 (STEMCELL Technologies) on different surfaces at 37°C and 5% CO2 in a humidified incubator. For each experiment cells were dissociated using ReLeSR^{™} (STEMCELL Technologies) for 3-5 min following the manufacturer's protocol. The resulting clumps were seeded onto the test plates and grown for 4 days. Human bone marrow derived MSC (hBM-MSC) were cultured in StemPro (StemPro^{™} MSC SFM, Gibco, #A1033201) media. Cells were seeded at a density of 5.000 cells/cm2 into 24-well plates and cultured until 90% confluency at 37°C and 5% CO₂ in a humidified incubator. After fixation with 4% PFA, cells were stained with 0.5% Crystal violet for 10-20 min, then washed and dried at room temperature. The crystal violet stain was subsequently released from the cells in order to quantify cell numbers using absorbance spectroscopy.

### FGF-2 concentration determination

The concentration of bFGF in mTESR^{™}1 after incubation for different time points at 37°C was determined using the ELISA kit for human bFGF (Sigma Aldrich, catalog no. RAB0182-1KT). Briefly, all components of the mTESR^{™} 1 kit were combined and incubated in 6-well plates that were previously coated with the coating and another animal-component free coating that does not contain NCP as a component. Plates were placed into a cell culture incubator for 24, 48, 72, and 96 hours at 37°C, 5% CO₂. The ELISA was performed according to the manufacturer's instructions using the provided protein standard. Each measure was run in duplicate and the experiment was performed in triplicate.

### 3D cultivation of mesenchymal stromal cells

Human bone marrow derived MSCs (hBM-MSC) were cultured in StemPro (StemPro^{™} MSC SFM, Gibco, #A1033201) media. Cells were seeded at a density of 4,000-5,000 cells/cm² into Erlenmeyer flasks containing 15 g/L coated or non-coated microcarriers. After a static attachment period overnight at 37°C and 5% CO2 in a humidified incubator, flasks were shaken at 90-110 rpm over the course of 11 days. Glucose consumption was measured using the BIOSEN C-line device (EKF diagnostics). Cells were detached by adding TrypLE^{™} Express Enzyme (Gibco^{™}) and Collagenase for 20 mins and counted using the Eve^{™} NanoEntek cell counter.

### II. Results

### Deposition of the layer-by-layer coating is driven by charged-based interactions

The coating of cell culture ware and scaffolds is carried out using the LbL approach. The driving force of this deposition process is electrostatic interaction¹⁷. Thus, charged molecules are particularly suitable for LbL processes. Here, a method is provided employing two classes of oppositely charged biofunctional components to build effective coatings - a positively charged peptide-polymer-conjugate (in the following referred to as "conjugate") and a negatively charged polysaccharide (NCP) (Figure 1A).

The conjugate consists of a 4-arm star-shaped PEG molecule, which acts as the structural backbone of the coating. To each of the four arms, a peptide sequence is coupled. The sequence consists of a linker peptide and a biofunctional peptide. The linker peptide consists of repetitive units of lysin (K) and alanine (A), generating a positive molecular charge. The linker sequence has been designed to connect effectively to NCP¹⁸. The biofunctional peptide provides cells with enhanced and selective attachment to the coating (see sequences list). It has been reported that by selection of linker and biofunctional peptide sequence, optimal cell attachment and growth is supported in a cell type specific manner^{13,15}.

The NCP is a highly sulfated polysaccharide molecule of 4 to 2 kDa in size, carrying a high density of negative charged moieties. Commonly, heparin has been used, a glycosaminoglycan (GAG) obtained as a naturally occurring product from different animal sources. Alternatively, synthetic dextran sulfate can be employed as animal-component free alternative, which qualifies the so-formed coating for applications in cell culture, regenerative medicine, and cellular agriculture.

Exploiting the interaction of the two components classes, coating strategies were developed based on the LbL approach to address various types of scaffolds with different surface charge. Figure 1B describes schematically applicable strategies for negative, positive, and neutral charged surfaces. The effectiveness of each of these strategies was proven on a model scaffold by zeta potential measurements. As model scaffolds, aqueous suspensions of polystyrene microbeads (d = 2-3 µm) with positive (top), negative (middle) or neutral surface charge (bottom) were used.

The effectiveness of the deposition was monitored by zeta potential measurements. The corresponding plots indicate that the surface charge alternates strongly with each coating step demonstrating that the LbL deposition of both NCP and conjugate is effective. For a positively charged microbead, the first coating layer is an NCP followed by a second layer of conjugate. A first layer of conjugate is applied on a negatively charged microbead, followed by a second layer of NCP. Consequently, the zeta potentials show a strong alternation with each coating step underlining the effectiveness of the proposed charged-based strategy.

In the case of a neutral surface, a first layer of a charge-inducing component, such as poly-D-lysine (PDL), can create a highly positive charge on the substrate. Poly-D-lysine is commonly used and known to be an effective material to coat tissue culture ware¹⁹. The PDL layer is followed by a second layer of NCP (layer Z) and a third layer of conjugate as previously proposed for positively charged surfaces (layer Y). The corresponding zeta potential measurement indicates that upon coating with PDL a positive surface charge is induced on the initially uncharged microbead. Consequently, this provides a starting point to enable an effective coating with NCP (layer Z) and conjugate (layer Y).

A positively charged scaffold is the most preferred base material for our LbL coating approach. It enables an effective deposition of NCPs, while the accessibility of the conjugate in a second coated layer promotes effective interaction of the cells with the biofunctional peptide sequences and thus, optimal cell attachment and proliferation of mesenchymal stromal cells (see next section).

Moreover, zeta potential measurements allow monitoring the evolution of the surface charge of positively and negatively charged microcarrier upon the stepwise deposition of coating material (Figure 2). In this experiment, increasing amounts of NCP were added stepwise to positively charged microcarriers. This results in a significant change in the zeta potential from positive to negative values which reaches a plateau at ~ -50 mV, indicating the saturation of the coating of the bead surface (Figure 2A). Accordingly, in the case of negatively charged microcarriers the stepwise addition of conjugate leads to an increase of the zeta potential until reaching a plateau (Figure 2B). The results clearly show that the driving force of our coating approach is a charge-based interaction.

To summarize, in this work, a charge-based strategy is provided to functionalize differently charged scaffolds with a non-covalent coating consisting of a conjugate and an NCP. This strategy shows broad versatility and can be monitored efficiently by zeta potential measurements.

### Natural microcarriers and scaffolds can be coated using a charged-based interaction strategy

Since the zeta-sizer technology cannot be applied to measure the surface charge of complex scaffolds or microcarriers with diameter above 100 µm as typically used in bioprocesses, we used fluorescently labeled components to visualize the deposition using fluorescence microscopy. Positively charged microcarriers were coated and imaged using a wide-field microscope (Figure 3A) to demonstrate that the previously described principle using zeta potential measurements is translatable with fluorescence microscopy. Carrying out a coating with the negatively charged polysaccharide (NCP) containing a fluorescent label leads to efficient deposition of NCPs on the microcarrier as seen by their bright fluorescence (Figure 3A NCP coated). The coating with the fluorescently labelled conjugate is also efficient in a second step after the NCP deposition. Accordingly, the coated microcarriers show a bright fluorescence (Figure 3A NCP + conj. coated).

In a second example natural starch-based microcarriers were used and the LbL deposition was monitored (Figure 3B). While the conjugate component is adsorbed on the surface of the starch microcarrier, the NCP component is not deposited (Figure 3B NCP + conj. coated). The application of a charge-inducing component (in this example PDL) enabled a stepwise application of the LbL coating (PDL, NCP, conjugate) on the starch microcarriers. Fluorescent signals from both NCP and conjugate were detected on each microcarrier after such treatment (Figure 3B PDL + NCP + conj. coated).

These experiments show that the LbL coating approach can be universally applied onto different types of natural scaffolds by introducing charge onto the surfaces.

In a further example, the use of fluorescently labelled coating components allows to determine the amount of deposited material on the microcarrier in a semi-quantitative manner. The resulting fluorescence intensity serves as a measure for the deposited amount of material and strongly depends on the concentration of the coating solution (Figure 4). Exemplified by positively charged microcarriers coated beforehand with NCP (Figure 4A) and negatively charged microcarriers (Figure 4B), the coating with fluorescently labelled conjugate solutions of increasing concentration results in an increase in the mean fluorescence intensity and thus, in the amount of conjugate deposited on the microcarrier. The results indicate a measurable deposition of coating material on positively charged surfaces in the range of 1 to 50 µM. Effective fluorescence signal on negatively charged microcarriers has been measured for the range of 5 to 50 µM.

### Modular characteristics of layer-by-layer coating enable optimal growth of stem cells

In the given examples the promotion of cell growth was evaluated in respect of the number and order of layers as well as the concentration of the coating material in an LbL approach. The promotion of cell growth of the LbL coating has been evaluated with induced pluripotent stem cells (iPSCs) and mesenchymal stromal cells (MSCs), two anchorage-dependent stem cell types widely used in regenerative medicine and cellular agriculture. To understand the impact of the number of components layered onto each other, MSCs were grown in 2D on standard surfaces (control) or on 2 and 3 layers of the LbL coating (Figure 5A) and the number of cells was determined at confluence. Both 2-layer coatings performed similar to the standard control, and there was no difference in overall cell growth found when either NCP is deposited 'outside' facing the cells or when the conjugate component faces towards cells. However, the 3-layer coating improved the cell growth by ~0.3-fold, indicating that adjustments in the number of layers can be beneficial for cell growth.

Next, the order of the 3-layer coating was alternated to investigate the impact of the outside layer on cell growth. Although the alternation in 2-layer coatings did not show a difference in cell growth, applying conjugate as last layer improved cell growth by ~0.25 fold for 3-layer coatings (Figure 5B). It should be noted that only few cells grow on the negative control (no coating), so the improved LbL (Conj-NCP-Conj) coating promotes cell growth 8-fold higher than control.

Neither alternation of layers nor the number of layers impacted the short-term growth (<5 days, one passage) of iPSCs in 2D (data not shown). However, modulation of the concentration of the two components showed a strong response of the iPSCs. Analogue to the MSC experiments, iPSCs were seeded onto LbL-coated surfaces in 2D with different coating concentrations. Their cell number was determined after 4 days of growth. Coating concentrations below 1.5 µM showed nearly no cell growth (Figure 5C). The cell growth improved 0.3-fold when 20 µM instead of 1.5 µM was used.

### Characteristics of layer-by-layer coating and specific biofunctional peptides enable culture of anchorage-dependent stem cells

A previous study has shown that an LbL coating comprising iterating layers of heparin and conjugate can be applied to cultivate human umbilical vein endothelial cells (HUVECs) ¹⁶. In the given study, heparin has been coupled covalently using silanization chemistry to provide a starting layer. The applied conjugate contained the biofunctional peptide sequence RGDSP. This is a valuable method for modification of glass surfaces but may not be suitable for polymer-based scaffold materials. Further, this approach may not be biological relevant for cell types used in cellular agriculture and regenerative medicine such as MSCs and iPSCs. To test this hypothesis, we coated a polystyrene-based cell culture article with a formulation optimized for iPSC (iPSC MATRIX) comprising of Dextran sulfate and SEQ ID 6 in a non-covalent LbL coating approach. The formulation of heparin and RGDSP-containing conjugate has been applied according to the reference. The number of cells was determined after 4 days. Cells grown on the iPSC MATRIX compared to the RGDSP-coating showed significant (p < 0.0001) enhanced cell numbers (Figure 6A). Analogous to the cell growth measurement, we observed at the time point when the cell reached confluency on the iPSC MATRIX, cells on the RGDSP-coating barely grew (Figure 6B). The remaining cells on the RGDSP-coating had a compromised morphology and the edges of the colonies started to lift off (Figure 6C). Based on the morphology, it can be assumed that iPSC cannot survive long-term on the RGDSP-coating. In contrast, iPSC covered the iPSC MATRIX uniformly with expected dense cell morphologies indicating optimal growing conditions.

These results show that the LbL is well suited for the culture of stem cells and superior to existing coatings utilizing the combination of conjugate and NCP. The modulatory capacities of the LbL coating allow for improving cell performance and tailoring coatings towards cell type specific needs.

### The incorporation of NCPs component is beneficial for the stabilization of growth factors important for stem cell growth

The difference in cell growth on 3 layers with different components facing towards the cells raises the question about the impact of the NCP component on cells. The ability to stabilize growth factors by NCP components alone, e.g. Heparin, has been shown in vitro ²⁰. Cell growth, proliferation and differentiation relies on binding of growth factors present in cell culture media. Many cell types, particularly iPSCs, require the presence of high concentrations (<0.05 mg/L) of basic fibroblast growth factor (bFGF or FGF-2) to ensure their maintenance. Those high concentrations are important for cell growth, sternness maintenance and proliferation but are also used since the short half-life of bFGF in cell culture medium reduces their availability rapidly. The half-life of bFGF is also one of the major reasons that iPSC culture generally requires daily media change, a problematic making iPSC culture labor intensive.

To investigate the impact of an NCP component in the coatings on the half-life of bFGF, we measured the bFGF concentration in mTESR1^{™} medium over a course of 4 days. Normal mammalian cell culture condition was mimicked in a humidified chamber at 37°C with 5% CO₂. The concentration of bFGF was measured at each time point for an NCP-containing and a non-NCP-containing coating (Figure 7). As expected, within 24 hours the bFGF concentration decreases from 0.38 µg/mL to 0.063 µg/mL on the non-NCP containing coating. In contrast, the media incubated on the NCP-coating contained 0.3 µg/mL bFGF after 24 hours, significantly more than the non-NCP coating (p=0.0236, unpaired t-test). The bFGF concentration was also significantly higher at all subsequent time points compared to the non-NCP coating. Using non-linear regression, a one phase decay model (Y=(Y0 - Plateau)^{∗}exp(-K^{∗}X) + Plateau) was fitted to understand the stabilization benefit from the coating containing NCP. The concentration of bFGF on the non-NCP coating after 24 hours is reached on the coating only after 75 hours. The presence of NCP triples the lifetime of bFGF in standard cell culture media and shows that an NCP-containing coating can significantly stabilize growth factors important for mammalian cell culture like bFGF and is therefore an important component in our LbL coating approach.

### Exemplified 3D culture with mesenchymal stromal cells

Mesenchymal stromal cells are anchorage-dependent and microcarriers are a frequently chosen 3D scaffold to fulfill this requirement in industrial scale cell culture. Aggregate culture has been attempted but appeared to be most successful for lineage-specific differentiation or to improve functional outcomes ^{21,22}. In order to investigate the efficiency of the LbL coating approach, polystyrene microcarriers were coated with 2- and 3-layers and employed for the cultivation of MSC in shaker flasks over 11 days in serum-free media. Surface charge modified polystyrene microcarriers were used as control. The glucose consumption of the cells on microcarriers was monitored over time and used to determine whether media replenishment was necessary (Figure 8A). At the end of the cultivation period the fold expansion of each condition was determined based on input and output cell numbers (Figure 8B). Non-coated microcarriers did not support MSC growth in 3D culture. The 2-layer coating allowed for a 5-fold expansion in 3D culture over 11 days. As deduced from the previously shown 2D experiments, the 3-layer coating (Conj-NCP-Conj) resulted in the highest fold expansion, up to ~7-fold, in 3D culture. The 3-layer LbL coating showed large clumps of cells at the end of the culture period (Figure 8C) and nearly no beads without cells compared to the 2-layer LbL coating. These experiments show that the LbL coating approach provide relevant results for expansion of stem cells on microcarriers in serum-free media.

### III. Discussion

A chemically defined, animal-component free coating method is provided, that is simple in use, applicable for the culture of cells *in vitro* on plates, and synthetic and natural scaffolds for use in large-scale cell manufacturing, regenerative medicine, and cellular agriculture. The coating material is based on two components, a PEG-peptide-conjugate containing a biofunctional moiety, and a negatively charged polysaccharide (NCP). The conjugate provides the cell interactive construct, which is physically crosslinked by the NCP that further stabilizes mammalian cell culture's growth factors. As the interaction of the individual components is based on charge our invention is universally applicable for all types of scaffolds and allows for transferring cell culture procedures from 2D to 3D, from research scale to industrial scale.

### References

1. Jin, Y. L., Hafner, J., Dragieva, G. & Burg, G. Bioreactor Microcarrier Cell Culture System (Bio-MCCS) for large-scale production of autologous melanocytes. Cell Transplant. 13, 809-816 (2004).
2. Bancel, S. & Hu, W. S. Confocal laser scanning microscopy examination of cell distribution in macroporous microcarriers. Biotechnol. Prog. 12, 398-402 (1996).
3. Overstreet, M., Sohrabi, A., Polotsky, A., Hungerford, D. S. & Frondoza, C. G. Collagen microcarrier spinner culture promotes osteoblast proliferation and synthesis of matrix proteins. Vitr. Cell. Dev. Biol. - Anim. 39, 228-234 (2003).
4. Hillegas, W. J., Solomon, D. E. & Wuttke, G. H. Microcarrier beads having a styrene copolymer core and a covalently linked tri-methylamine exterior. Patent application US6214618B1 (1999).
5. Kleinman, H. K. et al. Basement Membrane Complexes with Biological Activity. Biochemistry 25, 312-318 (1986).
6. Hughes, C. S., Postovit, L. M. & Lajoie, G. A. Matrigel: a complex protein mixture required for optimal growth of cell culture. Proteomics 10, 1886-1890 (2010).
7. Terranova, V. P., Aumailley, M., Sultan, L. H., Martin, G. R. & Kleinman, H. K. Regulation of cell attachment and cell number by fibronectin and laminin. J. Cell. Physiol. 127, 473-479 (1986).
8. Ruoslahti, E. RGD and other recognition sequences for integrins. Annu. Rev. Cell Dev. Biol. 12, 697-715 (1996).
9. Mouw, J. K., Ou, G. & Weaver, V. M. Extracellular matrix assembly: A multiscale deconstruction. Nature Reviews Molecular Cell Biology vol. 15 771-785 (2014).
10. Afratis, N. et al. Glycosaminoglycans: Key players in cancer cell biology and treatment. FEBS J. 279, 1177-1197 (2012).
11. Pickford, C. E. et al. Specific glycosaminoglycans modulate neural specification of mouse embryonic stem cells. Stem Cells 29, 629-640 (2011).
12. Capila, I. & Linhardt, R. J. Heparin - Protein interactions. Angewandte Chemie - International Edition vol. 41 390-412 (2002).
13. Wieduwild, R. et al. Coacervation-Mediated Combinatorial Synthesis of Biomatrices for Stem Cell Culture and Directed Differentiation. Adv. Mater. 30, 1706100 (2018).
14. Zhang, Y., Thomas, A., Wetzel, R., Husman, D. & Wieduwild, R. Non-covalently assembled biomatrix layer. (2017).
15. Thamm, K. et al. A Novel Synthetic, Xeno-Free Biomimetic Surface for Serum-Free Expansion of Human Mesenchymal Stromal Cells. Adv. Biosyst. 4, 2000008 (2020).
16. Thomas, A. K. et al. Layer-by-Layer Assembly of Heparin and Peptide-Polyethylene Glycol Conjugates to Form Hybrid Nanothin Films of Biomatrices. ACS Appl. Mater. Interfaces 10, 14264-14270 (2018).
17. Sukhorukov, G. B. et al. Layer-by-layer self assembly of polyelectrolytes on colloidal particles. Colloids Surfaces A Physicochem. Eng. Asp. 137, 253-266 (1998).
18. Wieduwild, R. et al. Minimal peptide motif for non-covalent peptide-heparin hydrogels. J. Am. Chem. Soc. 135, 2919-2922 (2013).
19. Mazia, D., Schatten, G. & Sale, W. Adhesion of cells to surfaces coated with polylysine. Applications to electron microscopy. J. Cell Biol. 66, 198-200 (1975).
20. Flaumenhaft, R., Moscatelli, D. & Rifkin, D. B. Heparin and heparan sulfate increase the radius of diffusion and action of basic fibroblast growth factor. J. Cell Biol. 111, 1651-1659 (1990).
21. Jossen, V. et al. Mass Production of Mesenchymal Stem Cells - Impact of Bioreactor Design and Flow Conditions on Proliferation and Differentiation. Cells Biomater. Regen. Med. (2014) doi: 10.5772/59385.
22. Sart, S., Tsai, A. C., Li, Y. & Ma, T. Three-dimensional aggregates of mesenchymal stem cells: Cellular mechanisms, biological properties, and applications. Tissue Eng. - Part B Rev. 20, 365-380 (2014).

## Claims

1. A coated article for culturing primary cells and stem cells, said coated article comprising a cell culture scaffold material with a non-covalent coating, said non-covalent coating comprises or consists of one or more layers selected from a layer consisting of at least one negatively charged polysaccharide (NCP), a layer consisting of a positively charged conjugate and optionally a layer comprising or consisting of a charge-inducing component, wherein said charge-inducing component of said primer layer is selected from poly-L-lysine, poly-D-lysine, poly-ornithine, poly(allylamine hydrochloride) (PAH), polydimethyldiallyl ammonium chloride (PDDA), and positively charged polypeptides; and wherein the surface of the coated article shows a surface charge in the range between -50 mV and 50 mV, preferably between -30 mV and 30 mV, more preferably between -10 mV and 10 mV, most preferably between -3 mV and 3 mV.

2. The coated article of claim 1, wherein the cell culture scaffold material is selected from synthetic polymers such as polystyrene and poly-lactic-glycolic acid (PLGA), and natural polymers such as polysaccharides like dextran, starch, alginate, pectin, proteinergic material like silk, soy protein, collagen, and gelatin.

3. The coated article according to any one of the preceding claims, wherein said cell culture scaffold material comprises a negatively charged surface or positively charged surface, which comprises a multilayer coating comprising or consisting of the structure (YZ)ₙ or (YZY)ₙ for the negatively charged surface; or (ZY)ₙ or (ZYZ)ₙ for the positively charged surface, wherein
Y represents a layer comprising or consisting of a positively charged conjugate;
Z represents a layer comprising or consisting of at last one negatively charged polysaccharide (NCP); and
n is an integer between 1 and 100.

4. The coated article according to claim 1 or 2, wherein said cell culture scaffold material comprises a neutral (uncharged) surface, which comprises a coating comprising or consisting of
i. a layer comprising or consisting of a charge-inducing component as primer layer; and
ii. a multilayer coating comprising or consisting of the structure (ZY)ₙ or (ZYZ)ₙ as defined in claim 4.

5. The coated article according to any one of the preceding claims, wherein said cell culture scaffold material has a three-dimensional structure, such as a microcarrier.

6. The coated article according to any one of the preceding claims, wherein said negatively charged polysaccharide (NCP) is poly(sodium-4-styrenesulfonate) (PSS) or a sulfated or phosphorylated oligosaccharide, preferably selected from a group consisting of polysaccharides which comprises heparin, heparan sulfate, chondroitin sulfate, dermatan sulfate and keratin sulfate, dextran sulfate, α-cyclodextrin sulfate, β-cyclodextrin sulfate, γ-cyclodextrin sulfate, α-cyclodextrin phosphate, β-cyclodextrin phosphate, and γ-cyclodextrin phosphate.

7. The coated article according to any one of the preceding claims, wherein said positively charged conjugate consists of a PEG molecule, wherein a peptide sequence is coupled to each of the ends of said PEG, and wherein said peptide sequence consists of a linker peptide (KA)ₙ and a biofunctional peptide, wherein said (KA)ₙ, K is lysine, A is alanine and n is an integer selected from 3, 4, 5, 6, 7, 8, 9, 10, 11, and 12, and wherein said biofunctional peptide is a peptide selected from the group consisting of SEQ ID NOs: 4 to 344.

8. The coated article according to any one of the preceding claims, wherein said charge-inducing component of said primer layer is selected from poly-L-lysine, poly-D-lysine, poly-ornithine, poly(allylamine hydrochloride) (PAH), polydimethyldiallyl ammonium chloride (PDDA) und positively charged polypeptides.

9. The coated article according to any one of the preceding claims, wherein said coating of said coated article is obtainable by layer-by-layer coating, which is driven by charge-based interactions and/or adsorption between the surface of the cell culture scaffold material and the coating layers.

10. The coated article according to any one of the preceding claims, further comprising primary cells and/or stem cells.

11. A method for preparing a coated article comprising a cell culture scaffold material with a non-covalent coating according to any one of the preceding claims, comprising the steps of
i. selecting a cell culture scaffold material;
ii. measuring the charge (zeta potential) of the surface of the cell culture scaffold material, and
iii. depending on the charge of the surface of the cell culture scaffold, coating said cell culture scaffold with a multilayer coating comprising or consisting of the structure (YZ)ₙ or (YZY)ₙ for a negatively charged surface; or (ZY)ₙ or (ZYZ)ₙ for a positively charged surface, wherein
Y represents a layer comprising or consisting of a positively charged conjugate;
Z represents a layer comprising or consisting of at least one negatively charged polysaccharide (NCP); and
n is an integer in the range of 1 and 100.

12. The method according to claim 11, wherein said cell culture scaffold material comprises a neutral (uncharged) surface, which is coated with
a layer comprising or consisting of a charge-inducing component as primer layer; and a multilayer coating comprising or consisting of the structure (ZY)ₙ or (ZYZ)ₙ as defined in claim 11.

13. The method according to claim 11 or 12, further comprising the step of incubating the coated article in a culture medium comprising primary cells and/or stem cells, preferably of human origin.

14. Use of the coated article according to any one of the preceding claims in a method for culturing primary cells and/or stem cells.

15. The coated article according to any one of claims 1 to 10 for use in regenerative medicine, aesthetic dermatology, plastic surgery procedures and in cellular agriculture.
